# EUROPEAN PATENT APPLICATION

(11) **EP 4 778 940 A1**
(43) Date of publication of application: **22.07.2026**
(21) Application number: 24878898.6
(22) Date of filing: 11.10.2024
(51) Int. Cl.: C07K 16/28, C12N 15/85, C12N 15/13, C12N 5/10, C07K 19/00, G01N 33/574, G01N 33/68, A61K 39/395, A61K 39/00, A61P 35/00

(54) **ANTI-HUMAN HHLA2 NANOBODY AND USE THEREOF**

(30) Priority: 17.10.2023 CN 202311350915
(71) Applicant: Artivila (ShenZhen) Innovation Center, Ltd., Guangdong, 518172 (CN)
(72) Inventor: ZHU, Zhendong, Shenzhen, Guangdong 518172 (CN); NIU, Deqiang, Shenzhen, Guangdong 518172 (CN); LIN, Dong, Shenzhen, Guangdong 518172 (CN); CHEN, Liang, Shenzhen, Guangdong 518172 (CN); ZHANG, Qing, Shenzhen, Guangdong 518172 (CN); YANG, Qi, Shenzhen, Guangdong 518172 (CN)
(74) Representative: Buzzi, Notaro & Antonielli d'Oulx S.p.A.
(86) International application number: PCT/CN2024/124073
(87) International publication number: WO 2025/082254

(57) **Abstract**

Provided are an anti-human HHLA2 nanobody and a use thereof. The variable regions of the nanobody include CDR1, CDR2, and CDR3; the amino acid sequence of the CDR1 includes any one of the sequences shown in SEQ ID NOs: 29-36 or a mutation thereof; the amino acid sequence of the CDR2 includes any one of the sequences shown in SEQ ID NOs: 37-46 or a mutation thereof; and the amino acid sequence of the CDR3 includes any one of the sequences shown in SEQ ID NOs: 47-56 or a mutation thereof, wherein the mutation is a substitution, insertion or deletion of one, two, three or four amino acids in the amino acid sequence. The nanobody provided can specifically bind to tumor cells expressing human HHLA2, and can improve the killing effect of immune cells on tumor cells by blocking the HHLA2-KIR3DL3 signaling pathway.

## Description

### TECHNICAL FIELD

The present application belongs to the field of biopharmaceutical technology and relates to an anti-human HHLA2 nanobody and use thereof.

### BACKGROUND

Human endogenous retrovirus-H long terminal repeat-associating 2 (HHLA2) was discovered during the search for human retrovirus-long terminal repeats (LTRs) (Genomics, 1999, 59(3): 255-263). HHLA2 is also referred to as B7-H7 or B7y and is a newly identified molecule of the B7 family. Currently, 10 members of the B7 family have been identified: CD80 (B7-1), CD86 (B7-2), B7-H1 (PD-L1 or CD274), B7-DC (CD273 or PD-L2), B7-H2 (CD275), B7-H3 (CD276), B7-H4 (B7S1, B7x, or VTCN1), B7-H5 (Vista, GI24, or PD-1H), B7-H6 (NCR3LG1), and B7-H7 (HHLA2 or B7y) (Frontiers in Immunology, 2020, 11: 681).

HHLA2 is mainly distributed and highly expressed in various tumor tissues and is expressed only in small amounts in the epithelial cells of the intestine, kidney, gallbladder, breast, and placental trophoblast tissue. HHLA2 is closely related to the growth, metastasis, pathological grading, and prognosis of various tumors such as pancreatic cancer, lung cancer, breast cancer, osteosarcoma, colorectal cancer, and kidney cancer. In one aspect, HHLA2 can interact with TMIGD2 to co-stimulate T cells in the context of TCR-mediated activation and enhance T cell proliferation and cytokine production through an AKT-dependent signaling cascade. In the other aspect, HHLA2 can interact with KIR3DL3 on T/NK immune cells to recruit SHP-1 and SHP-2, thereby weakening downstream Vav1, ERK1/2, AKT, and NF-κB signaling. Thus, HHLA2 shows immunosuppressive activity (International Immunopharmacology, 2023, 121: 110403).

Cancer immunotherapy enhances the body's immunity against tumors by reducing immune escape. Compared with traditional chemotherapy, immunotherapy has the advantages of a small drug dosage, a wider scope of action, and a few side effects. For example, immune checkpoint inhibitors, such as anti-PD1 and anti-PD-L1, have achieved great success in clinical practice and show excellent anti-tumor activity. However, these immune checkpoint inhibitors have a low response rate (about 30%) in patients in clinical practice due to a complex comprehensive mechanism of a tumor microenvironment where the existing various immunosuppressive molecules, inhibitory cells, and special tumor tissue structures jointly block the occurrence of normal immune responses. The blocking of a single immune checkpoint often fails to achieve an ideal therapeutic effect. Existing preclinical studies have proved that HHLA2 and PD-L1 are independently distributed in tumor tissues, HHLA2 can promote the immune escape of tumors, and drugs (especially antibody drugs) that block the HHLA2/KIR3DL3 pathway can inhibit tumor development and metastasis and can produce a synergistic effect when used in combination with PD-(L)1 monoclonal antibodies. Therefore, anti-tumor therapy targeting HHLA2/KIR3DL3 has attracted much attention at present (Cancer immunology research, 2021, 9(2): 156-169; Science immunology, 2021, 6(61): eabf9792).

In the peripheral blood of animals such as alpacas and camels, an antibody is present which naturally lacks light chains and is only composed of heavy chains. A heavy chain contains two constant domains (CH2 and CH3), a hinge region, and a variable heavy chain domain (VHH, i.e., an antigen-binding region). The VHH has a relative molecular mass of about 15 kDa, which is only 1/10 of that of a conventional antibody. The antibody has a molecular height and diameter at a nanometer level and can penetrate the blood-brain barrier. Therefore, the antibody is referred to as a nanobody (Nb). The nanobody generally has high solubility, high stability (difficult to aggregate and resistant to denaturing conditions such as high temperature, strong acid, and strong alkali), high affinity, and relatively low immunogenicity (more than 80% homology with a human antibody) and is amenable to engineering, suitable for various prokaryotic and eukaryotic expression systems, and widely applied to the development of antibody drugs, detection reagents, etc. (Annual review of biochemistry, 2013, 82: 775-797; Analytical and Bioanalytical Chemistry, 2019, 411: 1703-1713). At present, most antibodies acting on the HHLA2/KIR3DL3 signaling pathway in public reports are hybridoma/murine/human monoclonal antibodies (CN114729052A, CN112153977A, WO2023138579A1, and US20210198366A1), where only HBM1020, a fully human monoclonal antibody developed by Harbour BioMed based on a self-developed H2L2 transgenic mouse platform, is in the clinical research stage (ClinicalTrials.gov ID: NCT05824663). There are no reports on the development progress of nanobody drugs targeting HHLA2.

### SUMMARY

The present application provides an anti-human HHLA2 nanobody and use thereof.

In a first aspect, the present application provides an anti-human HHLA2 nanobody, which has a variable region including CDR1, CDR2, and CDR3, wherein an amino acid sequence of CDR1 includes any one of the sequences shown in SEQ ID NO: 29 to SEQ ID NO: 36 or a mutant thereof, an amino acid sequence of CDR2 includes any one of the sequences shown in SEQ ID NO: 37 to SEQ ID NO: 46 or a mutant thereof, an amino acid sequence of CDR3 includes any one of the sequences shown in SEQ ID NO: 47 to SEQ ID NO: 56 or a mutant thereof, wherein the mutant refers to an amino acid sequence having a substitution, insertion, or deletion of 1, 2, 3, or 4 amino acids.
SEQ ID NO: 29: YYVIG.
SEQ ID NO: 30: STPMY.
SEQ ID NO: 31: DYSIG.
SEQ ID NO: 32: NYLMG.
SEQ ID NO: 33: FNVMG.
SEQ ID NO: 34: FTAAG.
SEQ ID NO: 35: FSAAG.
SEQ ID NO: 36: DYAIG.
SEQ ID NO: 37: CISSSTGSTYSADSVKG.
SEQ ID NO: 38: CISSSTGSTYSAQSVKG.
SEQ ID NO: 39: CISSSDGSTYSADSVKG.
SEQ ID NO: 40: IMAPGGYEDIADSVRG.
SEQ ID NO: 41: CISSTHGTTYYTDSVKD.
SEQ ID NO: 42: AINSAGYSTVYAESVKG.
SEQ ID NO: 43: LISSGGTANYADSVKG.
SEQ ID NO: 44: VIANGGHTNYADSVKG.
SEQ ID NO: 45: VITGGGHTNYADSVKG.
SEQ ID NO: 46: CITSSTNRTYYANSVKG.
SEQ ID NO: 47: RRSPGIEGWCALPFFFGS.
SEQ ID NO: 48: RRSPGIEGWCALPEFFGS.
SEQ ID NO: 49: RISPGIEGWCALPFFFGS.
SEQ ID NO: 50: RYSPGIEGWCALPFFFGS.
SEQ ID NO: 51: LNS.
SEQ ID NO: 52: GLDGLACHRRDSGSRYRTQLYDY.
SEQ ID NO: 53: RYDRWHDY.
SEQ ID NO: 54: TTPVGQY.
SEQ ID NO: 55: PPAFGS.
SEQ ID NO: 56: EPILCVLWSAGGAMDY.

The nanobody disclosed in the present application can specifically bind to tumor cells expressing human HHLA2 and can enhance a killing effect of immune cells on tumor cells by blocking the HHLA2/KIR3DL3 signaling pathway. Therefore, the nanobody disclosed in the present application has a potential for use in the development of medicaments for tumor treatment, detection reagents, etc.

In the present application, the mutant may include those designed for antibody screening and engineering during preparation or use processes of the antibody, for example, relevant mutations made to potential post-translational modification (PTM) sites in the variable region (particularly CDRs), including potential asparagine deamidation sites (such as NG, NS, and NH), potential aspartate isomerization sites (such as DG, DP, and DS), potential N-glycosylation sites (N-{P}S/T), and potential oxidation-sensitive sites (M, W). The mutant may also include relevant mutations made to potential sites that affect properties of the antibody, such as aggregation, glycosylation, solubility, viscosity, expression level, activity (such as affinity), purity, isoelectric point, melting temperature (Tm), fragmentation, pharmacokinetics (PK), and immunogenicity.

Preferably, a mutant of CDR1 is the amino acid sequence shown in SEQ ID NO: 36 having 2 or 1 amino acid substitution selected from D1Y/G/N/S/T/R and S3A/V/G/S/T/W/Y.

Preferably, the amino acid sequence of CDR1 is the sequence shown in SEQ ID NO: 29 or SEQ ID NO: 31.

Preferably, a mutant of CDR2 is the amino acid sequence shown in SEQ ID NO: 39 having 2 or 1 amino acid substitution selected from D6T/E/V/R/L/A/I and D13Q/E.

Preferably, the amino acid sequence of CDR2 is the sequence shown in SEQ ID NO: 37 or SEQ ID NO: 38.

Preferably, a mutant of CDR3 in the heavy chain variable region is the amino acid sequence shown in SEQ ID NO: 47 having 2 or 1 amino acid substitution selected from R2I/Y/V/L/K/E/T/S and F14E/R/S/T.

Preferably, the amino acid sequence of CDR3 is the sequence shown in any one of SEQ ID NO: 48 to SEQ ID NO: 50.

The meanings of the above mutations are to be understood by those skilled in the art. For example, D13Q/E means that the amino acid D at position 13 in the amino acid sequence shown in SEQ ID NO: 39 is mutated to Q or E. The same applies to other amino acid substitutions such as D1Y/G/N/S/T/R, S3A/V/G/S/T/W/Y, D6T/E/V/R/L/A/I, R2I/Y/V/L/K/E/T/S, and F14E/R/S/T.

Preferably, the amino acid sequences of CDR1, CDR2, and CDR3 in the anti-human HHLA2 nanobody are the sequences shown in SEQ ID NO: 29, SEQ ID NO: 37, and SEQ ID NO: 47, respectively.

Preferably, the amino acid sequences of CDR1, CDR2, and CDR3 in the anti-human HHLA2 nanobody are the sequences shown in SEQ ID NO: 29, SEQ ID NO: 37, and SEQ ID NO: 48, respectively.

Preferably, the amino acid sequences of CDR1, CDR2, and CDR3 in the anti-human HHLA2 nanobody are the sequences shown in SEQ ID NO: 29, SEQ ID NO: 38, and SEQ ID NO: 47, respectively.

Preferably, the amino acid sequences of CDR1, CDR2, and CDR3 in the anti-human HHLA2 nanobody are the sequences shown in SEQ ID NO: 29, SEQ ID NO: 37, and SEQ ID NO: 49, respectively.

Preferably, the amino acid sequences of CDR1, CDR2, and CDR3 in the anti-human HHLA2 nanobody are the sequences shown in SEQ ID NO: 29, SEQ ID NO: 37, and SEQ ID NO: 50, respectively.

Preferably, the amino acid sequences of CDR1, CDR2, and CDR3 in the anti-human HHLA2 nanobody are the sequences shown in SEQ ID NO: 29, SEQ ID NO: 39, and SEQ ID NO: 47, respectively.

Preferably, the amino acid sequences of CDR1, CDR2, and CDR3 in the anti-human HHLA2 nanobody are the sequences shown in SEQ ID NO: 30, SEQ ID NO: 40, and SEQ ID NO: 51, respectively.

Preferably, the amino acid sequences of CDR1, CDR2, and CDR3 in the anti-human HHLA2 nanobody are the sequences shown in SEQ ID NO: 31, SEQ ID NO: 41, and SEQ ID NO: 52, respectively.

Preferably, the amino acid sequences of CDR1, CDR2, and CDR3 in the anti-human HHLA2 nanobody are the sequences shown in SEQ ID NO: 32, SEQ ID NO: 42, and SEQ ID NO: 53, respectively.

Preferably, the amino acid sequences of CDR1, CDR2, and CDR3 in the anti-human HHLA2 nanobody are the sequences shown in SEQ ID NO: 33, SEQ ID NO: 43, and SEQ ID NO: 54, respectively.

Preferably, the amino acid sequences of CDR1, CDR2, and CDR3 in the anti-human HHLA2 nanobody are the sequences shown in SEQ ID NO: 34, SEQ ID NO: 44, and SEQ ID NO: 55, respectively.

Preferably, the amino acid sequences of CDR1, CDR2, and CDR3 in the anti-human HHLA2 nanobody are the sequences shown in SEQ ID NO: 35, SEQ ID NO: 45, and SEQ ID NO: 55, respectively.

Preferably, the amino acid sequences of CDR1, CDR2, and CDR3 in the anti-human HHLA2 nanobody are the sequences shown in SEQ ID NO: 36, SEQ ID NO: 46, and SEQ ID NO: 56, respectively.

In the present application, the above-listed amino acid sequences of the CDRs are all represented according to the Kabat numbering system based on sequence variability (Kabat E A. Sequences of proteins of immunological interest [M]. US Department of Health and Human Services, Public Health Service, National Institutes of Health, 1991). As is known to those skilled in the art, the CDRs of the antibody may be represented according to various numbering systems in the art (such as IMGT, Chothia, Honegger, AbM, and Contact). Although the sequence scopes of the CDRs claimed in the present application are represented based on the Kabat numbering system, amino acid sequences defined according to other CDR numbering schemes also fall within the protection scope of the present application.

Preferably, the variable region in the nanobody further includes 4 framework regions.

Preferably, the nanobody includes a single-domain antibody, a VHH-Fc antibody, a bispecific antibody, or a multispecific antibody.

In the present application, the single-domain antibody (nanobody) refers to a VHH structure isolated or cloned from a heavy-chain antibody and is a smallest known unit capable of binding to a target antigen.

In the present application, the heavy-chain antibody (VHH-Fc antibody) refers to an antibody including a variable heavy chain domain (VHH), a hinge region (or a linker, such as a repeated "GGGGS (SEQ ID NO: 59)" amino acid sequence or a variant thereof), a constant domain CH2, and a constant domain CH3, and it is typically a homodimer.

In the present application, the term "bispecific antibody" or "multispecific antibody" covers an antibody having binding specificities for two or more epitopes. That is, the "bispecific antibody" or "multispecific antibody" has two or more single variable regions and thus can bind to different targets or different epitopes of the same target.

Preferably, the nanobody includes a variable region, a fragment crystallizable region of a heavy chain or a mutant thereof;
the mutant includes a mutation for enhancing/weakening the effector function of the fragment crystallizable region of the heavy chain or a mutation for prolonging or shortening a half-life of the fragment crystallizable region of the heavy chain, which is known to professionals in the art.

Preferably, the fragment crystallizable region of the heavy chain is selected from any one of human IgG1 or a mutant thereof, human IgG2 or a mutant thereof, human IgG3 or a mutant thereof, or human IgG4 or a mutant thereof;
or, the fragment crystallizable region of the heavy chain is selected from any one of murine IgG1 or a mutant thereof, murine IgG2a or a mutant thereof, murine IgG2b or a mutant thereof, or murine IgG3 or a mutant thereof.

Preferably, the fragment crystallizable region of the heavy chain is selected from any one of human IgG1 or a mutant thereof, or murine IgG2a or a mutant thereof.

Preferably, a source of the fragment crystallizable region of the heavy chain includes a human, a mouse, a rat, an alpaca, a goat, a rabbit, or a horse.

Preferably, an amino acid sequence of the variable region in the nanobody includes the sequence shown in any one of SEQ ID NO: 15 to SEQ ID NO: 28.
SEQ ID NO: 15: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARRSPGIEGWCALPFFFGS
WGQGTLVTVSS.
SEQ ID NO: 16: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARRSPGIEGWCALPEFFGS
WGQGTLVTVSS.
SEQ ID NO: 17: 
EVQLLESGGGLVQPGGSLRLSCAASGFTLDYYVIGWFRQAPGKEREGVSCISSSTGS
TYSAQSVKGRFTISRDTSKNTVYLQMNSLRAEDTAVYYCAARRSPGIEGWCALPFFFGS
WGQGTLVTVSS.
SEQ ID NO: 18: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARISPGIEGWCALPFFFGSW
GQGTLVTVSS.
SEQ ID NO: 19: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARYSPGIEGWCALPFFFGS
WGQGTLVTVSS.
SEQ ID NO: 20: 
DVQLQESGGGLVQPGGSLRLSCAASGFTLDYYVIGWFRQAPGKEREGVSCISSSDG
STYSADSVKGRFTISRDNAENTVYLQMNSLKPEDTAVYYCAARRSPGIEGWCALPFFFGS
WGQGTQVTVSS.
SEQ ID NO: 21: 
EVQLLESGGGLVQPGGSLRLSCAASGFTLDYYVIGWFRQAPGKEREGVSCISSSDGS
TYSADSVKGRFTISRDNSKNTVYLQMNSLRAEDTAVYYCAARRSPGIEGWCALPFFFGS
WGQGTLVTVSS.
SEQ ID NO: 22: 
DVQLQESGGGLVQPGGSLRLSCAASGFTFSSTPMYWYRQAPGKERDFVAIMAPGG
YEDIADSVRGRFSISRDNGNNTMYLQMNSLKPEDTALYYCRALNSWGQGTQVTVSS.
SEQ ID NO: 23: 
DVQLQESGGGLVQAGGSLRLSCAASGFPFDDYSIGWFRQAPGKAREGISCISSTHGT
TYYTDSVKDRFTITRDNAKNTVYLQMNNLKPEDAAVYYCAAGLDGLACHRRDSGSRY
 RTQLYDYWGQGTQVTVSS.
SEQ ID NO: 24: 
DVQLQESGGGLVQPGGSLRLSCAASGFTFSNYLMGWYRQAPGKERELVAAINSAG
YSTVYAESVKGRFTTSRDNAKSMVYLQMNSLKAEDTAVYYCNARYDRWHDYWGQGT
QVTVSS.
SEQ ID NO: 25: 
DVQLQESGGGLVQPGGSLRLSCAASGSRFSFNVMGWHRQAPGKQRELVALISSGGT
ANYADSVKGRFTISRDNAGNTMYLQMNTLKPEDTAVYYCTATTPVGQYWGQGTQVTV
SS.
SEQ ID NO: 26: 
DVQLQESGGGLVQPGGSLKLSCTASRRIFTFTAAGWYRQAPGQQREFVAVIANGGH
TNYADSVKGRFTISRDNAKNTVYLQMNSLKPEDTAVYYCNTPPAFGSWGPGTQVTVSS.
SEQ ID NO: 27: 
DVQLQESGGGLVQPGGSLRLSCTASTRIFTFSAAGWYRQAPGKQREFVAVITGGGH
TNYADSVKGRFTISRDNAKDTVYLQMNSLKPEDTAVYYCNTPPAFGSWGPGTQVTVSS.
SEQ ID NO: 28:

DVQLQESGGGLVQTGGSLRLSCAAPGFTLDDYAIGWFRQAPGKERERISCITSSTNR
TYYANSVKGRFTISRDSAKNTVYLQMNSLKPEDTAVYYCAAEPILCVLWSAGGAMDYW
GKGTQVTVSS.

Preferably, an amino acid sequence of the VHH-Fc antibody includes the sequence shown in any one of SEQ ID NO: 1 to SEQ ID NO: 14.
SEQ ID NO: 1: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARRSPGIEGWCALPFFFGS
WGQGTLVTVSSGGGGSDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIAVEWE
SNGQPENNYKTTPPVLDSDGSFFLYSRLTVDKSRWQQGNVFSCSVMHEALHNHYTQKS
LSLSPGK.
SEQ ID NO: 2: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARRSPGIEGWCALPEFFGS
WGQGTLVTVSSGGGGSDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEYKC
 
KVSNKALPAPIEKTISKAKGQPREPQVYTLPPSRDELTKNQVSLTCLVKGFYPSDIAVEWE
SNGQPENNYKTTPPVLDSDGSFFLYSRLTVDKSRWQQGNVFSCSVMHEALHNHYTQKS
LSLSPGK.
SEQ ID NO: 3: 
EVQLLESGGGLVQPGGSLRLSCAASGFTLDYYVIGWFRQAPGKEREGVSCISSSTGS
TYSAQSVKGRFTISRDTSKNTVYLQMNSLRAEDTAVYYCAARRSPGIEGWCALPFFFGS
WGQGTLVTVSSGGGGSDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIAVEWE
SNGQPENNYKTTPPVLDSDGSFFLYSRLTVDKSRWQQGNVFSCSVMHEALHNHYTQKS
LSLSPGK.
SEQ ID NO: 4: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARISPGIEGWCALPFFFGSW
GQGTLVTVSSGGGGSDKTHTCPPCPAPEAAGGPSVFLFPPKPKDTLMISRTPEVTCVVVD
VSHEDPEVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIAVEWES
NGQPENNYKTTPPVLDSDGSFFLYSRLTVDKSRWQQGNVFSCSVMHEALHNHYTQKSL
SLSPGK.
SEQ ID NO: 5: 
EVQLLESGGGLVQPGGSLRLSCAASGFTLDYYVIGWFRQAPGKEREGVSCISSSTGS
TYSADSVKGRFTISRDTSKNTVYLQMNSLRAEDTAVYYCAARYSPGIEGWCALPFFFGS
WGQGTLVTVSSGGGGSDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDIAVEWE
SNGQPENNYKTTPPVLDSDGSFFLYSRLTVDKSRWQQGNVFSCSVMHEALHNHYTQKS
LSLSPGK.
SEQ ID NO: 6: 
DVQLQESGGGLVQPGGSLRLSCAASGFTLDYYVIGWFRQAPGKEREGVSCISSSDG
STYSADSVKGRFTISRDNAENTVYLQMNSLKPEDTAVYYCAARRSPGIEGWCALPFFFGS
WGQGTQVTVSSGGGGSDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVE
WESNGQPENNYKTTPPVLDSDGSFFLYSRLTVDKSRWQQGNVFSCSVMHEALHNHYTQ
 KSLSLSPGK.
SEQ ID NO: 7: 
DVQLQESGGGLVQPGGSLRLSCAASGFTLDYYVIGWFRQAPGKEREGVSCISSSDG
STYSADSVKGRFTISRDNAENTVYLQMNSLKPEDTAVYYCAARRSPGIEGWCALPFFFGS
WGQGTQVTVSSGGGGSGPTIKPCPPCKCPAPNLLGGPSVFIFPPKIKDVLMISLSPIVTCV
VVDVSEDDPDVQISWFVNNVEVHTAQTQTHREDYNSTLRVVSALPIQHQDWMSGKEFK
CKVNNKDLPAPIERTISKPKGSVRAPQVYVLPPPEEEMTKKQVTLTCMVTDFMPEDIYVE
WTNNGKTELNYKNTEPVLDSDGSYFMYSKLRVEKKNWVERNSYSCSVVHEGLHNHHT
TKSFSRTPGK.
SEQ ID NO: 8: 
DVQLQESGGGLVQPGGSLRLSCAASGFTFSSTPMYWYRQAPGKERDFVAIMAPGG
YEDIADSVRGRFSISRDNGNNTMYLQMNSLKPEDTALYYCRALNSWGQGTQVTVSSGG
GGSGPTIKPCPPCKCPAPNLLGGPSVFIFPPKIKDVLMISLSPIVTCVVVDVSEDDPDVQIS
WFVNNVEVHTAQTQTHREDYNSTLRVVSALPIQHQDWMSGKEFKCKVNNKDLPAPIER
TISKPKGSVRAPQVYVLPPPEEEMTKKQVTLTCMVTDFMPEDIYVEWTNNGKTELNYK
NTEPVLDSDGSYFMYSKLRVEKKNWVERNSYSCSVVHEGLHNHHTTKSFSRTPGK.
SEQ ID NO: 9: 
DVQLQESGGGLVQAGGSLRLSCAASGFPFDDYSIGWFRQAPGKAREGISCISSTHGT
TYYTDSVKDRFTITRDNAKNTVYLQMNNLKPEDAAVYYCAAGLDGLACHRRDSGSRY
RTQLYDYWGQGTQVTVSSGGGGSGPTIKPCPPCKCPAPNLLGGPSVFIFPPKIKDVLMISL
SPIVTCVVVDVSEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQVTLTCMVTDF
MPEDIYVEWTNNGKTELNYKNTEPVLDSDGSYFMYSKLRVEKKNWVERNSYSCSVVH
EGLHNHHTTKSFSRTPGK.
SEQ ID NO: 10: 
DVQLQESGGGLVQPGGSLRLSCAASGFTFSNYLMGWYRQAPGKERELVAAINSAG
YSTVYAESVKGRFTTSRDNAKSMVYLQMNSLKAEDTAVYYCNARYDRWHDYWGQGT
QVTVSSGGGGSGPTIKPCPPCKCPAPNLLGGPSVFIFPPKIKDVLMISLSPIVTCVVVDVSE
DDPDVQISWFVNNVEVHTAQTQTHREDYNSTLRVVSALPIQHQDWMSGKEFKCKVNN
KDLPAPIERTISKPKGSVRAPQVYVLPPPEEEMTKKQVTLTCMVTDFMPEDIYVEWTNN
GKTELNYKNTEPVLDSDGSYFMYSKLRVEKKNWVERNSYSCSVVHEGLHNHHTTKSFS
RTPGK.
SEQ ID NO: 11: DVQLQESGGGLVQPGGSLRLSCAASGSRFSFNVMGWHRQAPGKQRELVALISSGGT 
ANYADSVKGRFTISRDNAGNTMYLQMNTLKPEDTAVYYCTATTPVGQYWGQGTQVTV
SSGGGGSGPTIKPCPPCKCPAPNLLGGPSVFIFPPKIKDVLMISLSPIVTCVVVDVSEDDPD
VQISWFVNNVEVHTAQTQTHREDYNSTLRVVSALPIQHQDWMSGKEFKCKVNNKDLPA
PIERTISKPKGSVRAPQVYVLPPPEEEMTKKQVTLTCMVTDFMPEDIYVEWTNNGKTEL
NYKNTEPVLDSDGSYFMYSKLRVEKKNWVERNSYSCSVVHEGLHNHHTTKSFSRTPGK.
SEQ ID NO: 12: 
DVQLQESGGGLVQPGGSLKLSCTASRRIFTFTAAGWYRQAPGQQREFVAVIANGGH
TNYADSVKGRFTISRDNAKNTVYLQMNSLKPEDTAVYYCNTPPAFGSWGPGTQVTVSSG
GGGSGPTIKPCPPCKCPAPNLLGGPSVFIFPPKIKDVLMISLSPIVTCVVVDVSEDDPDVQI
SWFVNNVEVHTAQTQTHREDYNSTLRVVSALPIQHQDWMSGKEFKCKVNNKDLPAPIE
RTISKPKGSVRAPQVYVLPPPEEEMTKKQVTLTCMVTDFMPEDIYVEWTNNGKTELNYK
NTEPVLDSDGSYFMYSKLRVEKKNWVERNSYSCSVVHEGLHNHHTTKSFSRTPGK.
SEQ ID NO: 13: 
DVQLQESGGGLVQPGGSLRLSCTASTRIFTFSAAGWYRQAPGKQREFVAVITGGGH
TNYADSVKGRFTISRDNAKDTVYLQMNSLKPEDTAVYYCNTPPAFGSWGPGTQVTVSSG
GGGSGPTIKPCPPCKCPAPNLLGGPSVFIFPPKIKDVLMISLSPIVTCVVVDVSEDDPDVQI
SWFVNNVEVHTAQTQTHREDYNSTLRVVSALPIQHQDWMSGKEFKCKVNNKDLPAPIE
RTISKPKGSVRAPQVYVLPPPEEEMTKKQVTLTCMVTDFMPEDIYVEWTNNGKTELNYK
NTEPVLDSDGSYFMYSKLRVEKKNWVERNSYSCSVVHEGLHNHHTTKSFSRTPGK.
SEQ ID NO: 14:

DVQLQESGGGLVQTGGSLRLSCAAPGFTLDDYAIGWFRQAPGKERERISCITSSTNR
TYYANSVKGRFTISRDSAKNTVYLQMNSLKPEDTAVYYCAAEPILCVLWSAGGAMDYW
GKGTQVTVSSGGGGSGPTIKPCPPCKCPAPNLLGGPSVFIFPPKIKDVLMISLSPIVTCVVV
DVSEDDPDVQISWFVNNVEVHTAQTQTHREDYNSTLRVVSALPIQHQDWMSGKEFKCK
VNNKDLPAPIERTISKPKGSVRAPQVYVLPPPEEEMTKKQVTLTCMVTDFMPEDIYVEW
TNNGKTELNYKNTEPVLDSDGSYFMYSKLRVEKKNWVERNSYSCSVVHEGLHNHHTT
KSFSRTPGK.

In the present application, the above-listed amino acid sequences of the CDRs are all represented according to the Kabat numbering system based on sequence variability (Kabat E A. Sequences of proteins of immunological interest [M]. US Department of Health and Human Services, Public Health Service, National Institutes of Health, 1991). As is known to those skilled in the art, the CDRs of the antibody may be represented according to various numbering systems in the art (such as IMGT, Chothia, Honegger, AbM, and Contact). Although the sequence scopes of the CDRs claimed in the present application are represented based on the Kabat numbering system, amino acid sequences defined according to other CDR numbering schemes also fall within the protection scope of the present application.

In a second aspect, the present application provides a nucleic acid molecule. The nucleic acid molecule encodes the anti-human HHLA2 nanobody according to the first aspect.

The nucleic acid may be prepared using conventional molecular biology techniques in the art. For example, the nucleic acid encoding the anti-human HHLA2 nanobody is obtained through molecular cloning, or the nucleic acid is obtained through codon optimization and artificial full-sequence synthesis. As is known to those skilled in the art, a base sequence encoding an amino acid sequence of the anti-human HHLA2 nanobody may form a polynucleotide homolog by appropriately introducing a substitution, a deletion, or an insertion. The homolog of the polynucleotide in the present application may be obtained through substitutions, deletions, or insertions of one or more bases of the gene encoding the anti-human HHLA2 nanobody, with the activity of the antibody maintained.

In a third aspect, the present application provides an expression vector. The expression vector includes the nucleic acid molecule according to the second aspect.

The expression vector may be obtained using conventional molecular biology techniques in the art. That is, the nucleic acid of the present application is inserted into various expression vectors by means such as enzyme digestion and ligation or homologous recombination to construct the expression vector.

In a fourth aspect, the present application provides a host cell. The host cell includes the nucleic acid molecule according to the second aspect or the expression vector according to the third aspect.

Host cells suitable in the present application include prokaryotic cells and/or eukaryotic cells. Commonly used prokaryotic host cells include *Escherichia coli* (such as TG1, HB2151, BL21 (DE3), and Rosetta (DE3)), *Agrobacterium*, and *Bacillus subtilis*. Commonly used eukaryotic host cells include yeast cells, insect cells, human embryonic kidney epithelial cells (such as HEK293, 293F, and 293T), and Chinese hamster ovary cells (such as CHOS and CHO-K1). Preferably, the prokaryotic host cell is *Escherichia coli* BL21 (DE3) or Rosetta (DE3) (for expressing the nanobody), and the eukaryotic host cell is HEK293 cells or CHO cells (for expressing the heavy-chain antibody).

In a fifth aspect, the present application provides a chimeric antigen receptor. The chimeric antigen receptor includes the anti-human HHLA2 nanobody according to the first aspect.

In a sixth aspect, the present application provides a genetically modified cell. The genetically modified cell includes the chimeric antigen receptor according to the fifth aspect.

Preferably, the genetically modified cell is a human immune cell.

Preferably, the human immune cell includes T cells, NK cells, or macrophages.

In a seventh aspect, the present application provides a bispecific or multispecific antibody. The bispecific or multispecific antibody includes the anti-human HHLA2 nanobody according to the first aspect.

In the present application, the bispecific or multispecific antibody includes the anti-human HHLA2 nanobody according to the first aspect of the present application and an antibody having another antigen-binding property and functionally linked to the nanobody. Another antibody includes, but is not limited to, an antibody or antigen-binding fragment capable of binding to a target/antigen such as PD1, PD-L1, VEGF, VEGFR1, VEGFR2, HER2, EGFR, CD73, Trop2, Trop1, Nectin-4, MSLN, TIGIT, CD3, CD28, CD80, LIV-1, GLP-1, c-Met, LILRB4, LILRB2, GD2, APOE4, MICA, Claudin18.2, CD20, AXL, CD47, CD19, 4-1BB, MUC16, or Siglec10.

In an eighth aspect, the present application provides a conjugate. The conjugate includes the anti-human HHLA2 nanobody according to the first aspect.

In the present application, the conjugate is formed by conjugating the nanobody of the present application to a functional agent.

The functional agent may be a cytotoxic agent such as a chemotherapeutic agent, a toxin (such as an enzymatically active toxin of bacterial, fungal, plant, or animal origin or a fragment thereof), or a radioactive isotope (radioactive conjugate), an antibiotic, a nucleolytic enzyme, or any combination thereof. Chemotherapeutic agents may be used to produce immunoconjugates, such as methotrexate, adriamicin, vinca alkaloid (vincristine), vinblastine, etoposide, doxorubicin, melphalan, mitomycin C, chlorambucil, daunorubicin, or other intercalating agents and enzymes and/or fragments thereof, such as nucleolytic enzymes, antibiotics, and toxins such as micromolecular toxins or enzymatically active toxins of bacterial, fungal, plant, or animal origin, including fragments and/or variants thereof. Suitable enzymatically active toxins and fragments thereof include, for example, diphtheria toxin A chain, non-binding active fragments of diphtheria toxin, exotoxin A chain (from *Pseudomonas aeruginosa*), ricin A chain, abrin A chain, modeccin A chain, alpha-sarcin, *Aleurites fordii* protein, Dianthin protein, *Phytolacca americana* protein (PAP I, PAP II, and PAP-S), momordica charantia inhibitor, curcin, crotin, *saponaria officinalis* inhibitor, gelonin, mitogellin, restrictocin, phenomycin, enomycin, and trichothecenes. Any appropriate radioactive nucleotide or radioactive reagent known or available in the art may be used to produce radioactively conjugated antibodies.

An immunoconjugate may be a complex formed by directly or indirectly conjugating the nanobody of the present application to a detectable moiety.

Detectable moieties include, but are not limited to, enzymes, prosthetic groups, fluorescent materials, luminescent materials, bioluminescent materials, radioactive materials, positron-emitting metals, and non-radioactive paramagnetic metal ions.

Fluorescently labeled compounds suitable in the present application include fluorescein isothiocyanate, rhodamine, phycoerythrin, phycocyanin, allophycocyanin, ortho-phthalaldehyde, and fluorescamine.

Chemiluminescently labeled compounds suitable for the present application include luminol, isoluminol, aromatic acridine esters, imidazole, acridine salts, and oxalic esters.

Bioluminescent compounds suitable for the present application include luciferin, luciferase, and aequorin.

Enzymes suitable in the present application include horseradish peroxidase, alkaline phosphatase, glucose oxidase, β-D-galactosidase, urease, catalase, or glucoamylase.

Labeling for the purposes of detection and/or analysis and/or diagnosis depends on the use of particular detection/analysis/diagnostic techniques and/or methods, such as immunohistochemical staining of (tissue) samples, flow cytometry, laser scanning cytometry detection, fluoroimmunoassay, enzyme-linked immunosorbent assay (ELISA), radioimmunoassay (RIA), bioassay (such as phagocytosis assay), and Western blotting application. Labels suitable for detection/analysis/diagnostic techniques and/or methods known in the art are known to those skilled in the art.

In a ninth aspect, the present application provides a pharmaceutical composition. The pharmaceutical composition includes the anti-human HHLA2 nanobody according to the first aspect.

The pharmaceutical composition of the present application further includes a pharmaceutically acceptable carrier, including, but not limited to, various forms of carriers that have no side effects on the composition of the pharmaceutical composition and that have been approved by the Food and Drug Administration for use in humans or animals, such as any adjuvant, vector, excipient, glidant, sweetener, diluent, preservative, dye/colorant, flavor enhancer, surfactant, wetting agent, dispersing agent, suspending agent, stabilizer, isotonic agent, solvent, or emulsifier.

Generally, the pharmaceutical composition must be sterile and stable under the conditions of production and storage. The pharmaceutical composition may be formulated into a solution, a microemulsion, a liposome, or other ordered structure suitable for high medicament concentrations. The carrier may be a solvent or dispersing medium including, for example, water, ethanol, a polyol (such as glycerol, propylene glycol, or liquid polyethylene glycol), or a suitable mixture thereof. For example, proper fluidity may be maintained by using a coating such as lecithin to maintain a desired particle size in the case of dispersions and by using a surfactant. In many cases, it is preferable to include an isotonic agent in the composition, such as sugar, a polyol such as mannitol or sorbitol, or sodium chloride. Prolonged absorption of the injectable composition may be caused by including, in the composition, an agent that delays absorption, for example, monostearate or gelatin.

The pharmaceutical composition may be administered through oral administration, injection, nasal administration, percutaneous administration, or mucosal administration. The pharmaceutical composition may also include another anti-tumor antibody as an active ingredient.

The pharmaceutical composition may include, but is not limited to, liquid, frozen, and lyophilized compositions.

The pharmaceutical composition may be administered in combination with another medicament.

A dosage level of the pharmaceutical composition may be adjusted according to an amount of the composition required to achieve the desired diagnostic or therapeutic result. An administration regimen may be a single injection or multiple injections or may be adjusted. The selected dosage level and regimen are reasonably adjusted depending on various factors including the activity and stability, formulation, route of administration, and combination with another medicament or treatment, of the pharmaceutical composition, a disease or condition to be detected and/or treated, and a health status and a previous medical history of a subject to be treated. A therapeutically effective dosage of the pharmaceutical composition may be initially estimated in cell culture experiments or animal models such as rodents, rabbits, dogs, pigs, and/or non-human primates.

In a tenth aspect, the present application provides use of the anti-human HHLA2 nanobody according to the first aspect, the nucleic acid molecule according to the second aspect, the expression vector according to the third aspect, the bispecific or multispecific antibody according to the seventh aspect, or the conjugate according to the eighth aspect in preparation of a product for detecting human HHLA2 proteins.

In an eleventh aspect, the present application provides a product for detecting human HHLA2 proteins. The product includes the anti-human HHLA2 nanobody according to the first aspect, the chimeric antigen receptor according to the fifth aspect, the genetically modified cell according to the sixth aspect, the bispecific or multispecific antibody according to the seventh aspect, the conjugate according to the eighth aspect, or the pharmaceutical composition according to the ninth aspect.

The product includes products that detect antigen-antibody binding using ELISA, immunofluorescence detection, radioimmunoassay, luminescent immunoassay, colloidal gold immunochromatography, agglutination, and immunoturbidimetric assay.

A specific example of the product includes a kit. The kit further includes a solid-phase support. The antibody is immobilized on the solid-phase support (such as a microplate, a cover glass, or microbeads) or exists in a free state.

Further, the kit also includes a detectable moiety linkable to the antibody, where the detectable moiety is separably present in the kit; and/or a substrate corresponding to the detectable moiety; and/or enzyme-linked immunoassay reagents (including, but not limited to, a coating (buffer) solution, a washing (buffer) solution, a blocking solution, a fixing solution, a stop solution, and a color-developing solution); and/or instructions describing a method for detecting human HHLA2.

In a twelfth aspect, the present application provides use of the anti-human HHLA2 nanobody according to the first aspect, the nucleic acid molecule according to the second aspect, the expression vector according to the third aspect, the chimeric antigen receptor according to the fifth aspect, the genetically modified cell according to the sixth aspect, the bispecific or multispecific antibody according to the seventh aspect, the conjugate according to the eighth aspect, or the pharmaceutical composition according to the ninth aspect in preparation of a medicament for treating and/or preventing cancer.

The cancer includes, but is not limited to, melanoma, lung cancer, breast cancer, pancreatic cancer, Hodgkin's lymphoma, bladder cancer, gastric cancer, head and neck cancer, kidney cancer, prostate cancer, colorectal cancer, ovarian cancer, and blood cancer.

In a thirteenth aspect, the present application provides a method for detecting human HHLA2 proteins for a non-diagnostic purpose. The method includes subjecting a sample to contact with the anti-human HHLA2 nanobody according to the first aspect, the chimeric antigen receptor according to the fifth aspect, the genetically modified cell according to the sixth aspect, the bispecific or multispecific antibody according to the seventh aspect, the conjugate according to the eighth aspect, or the pharmaceutical composition according to the ninth aspect.

Preferably, the method further includes, after the contact, ELISA, western blotting, flow cytometry detection, or immunofluorescence detection.

Compared with the existing art, the present application has the following beneficial effects.

The present application provides a nanobody capable of specifically binding to human HHLA2, which has good anti-tumor activity. The nanobody disclosed in the present application can specifically bind to tumor cells expressing human HHLA2 and can enhance the killing effect of immune cells on tumor cells by blocking the HHLA2/KIR3DL3 signaling pathway. Therefore, the nanobody disclosed in the present application has a potential for use in the development of medicaments for tumor treatment, detection reagents, etc.

### BRIEF DESCRIPTION OF DRAWINGS

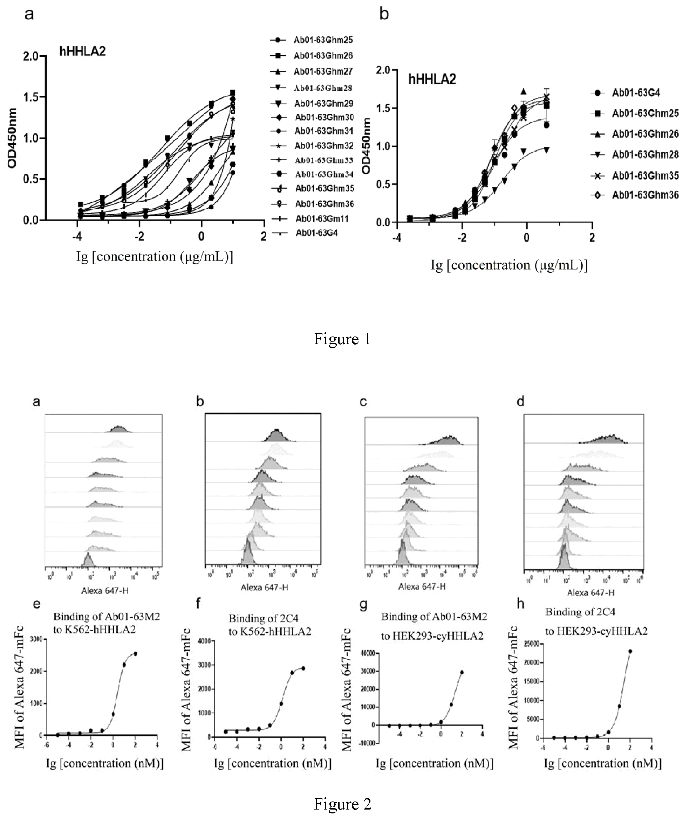

FIG. 1 shows ELISA results of the binding of some HHLA2 antibodies of the present application to human HHLA2, where FIG. 1a shows binding curves of a candidate parental antibody Ab01-63G4 and 13 humanized or engineered mutants thereof to human HHLA2, and FIG. 1b shows binding curves of the candidate parental antibody Ab01-63G4 and 5 humanized or engineered mutants thereof to human HHLA2.
FIG. 2 shows FACS results of the specific binding of some HHLA2 antibodies of the present application to human and cynomolgus monkey HHLA2, where FIG. 2a shows fluorescence signals when a candidate parental antibody Ab01-63M2 binds to K562 cells overexpressing human HHLA2 at different concentrations, FIG. 2b shows fluorescence signals when a positive control antibody 2C4 binds to K562 cells overexpressing human HHLA2 at different concentrations, FIG. 2c shows fluorescence signals when the candidate parental antibody Ab01-63M2 binds to HEK293 cells overexpressing cynomolgus monkey HHLA2 at different concentrations, FIG. 2d shows fluorescence signals when the positive control antibody 2C4 binds to HEK293 cells overexpressing cynomolgus monkey HHLA2 at different concentrations, FIG. 2e shows a binding curve of the candidate parental antibody Ab01-63M2 to K562 cells overexpressing human HHLA2, FIG. 2f shows a binding curve of the positive control antibody 2C4 to K562 cells overexpressing human HHLA2, FIG. 2g shows a binding curve of the candidate parental antibody Ab01-63M2 to HEK293 cells overexpressing cynomolgus monkey HHLA2, and FIG. 2h shows a binding curve of the positive control antibody 2C4 to HEK293 cells overexpressing cynomolgus monkey HHLA2.

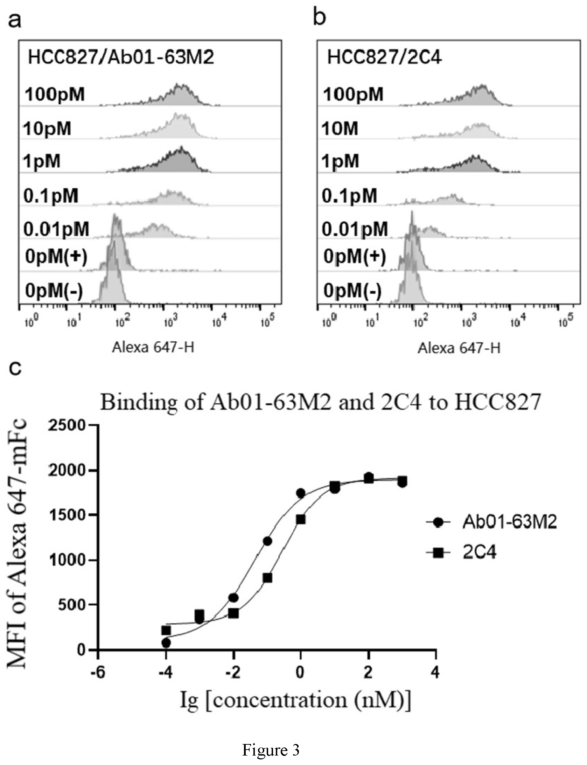

FIG. 3 shows FACS results of the binding of some HHLA2 antibodies of the present application to HCC827 tumor cells, where FIG. 3a shows fluorescence signals when a candidate parental antibody Ab01-63M2 binds to HCC827 cells (human non-small-cell lung carcinoma cells highly expressing HHLA2) at different concentrations, FIG. 3b shows fluorescence signals when a positive control antibody 2C4 binds to HCC827 cells at different concentrations, and FIG. 3c shows binding curves of the candidate parental antibody Ab01-63M2 and the positive control antibody 2C4 to HCC827 cells.

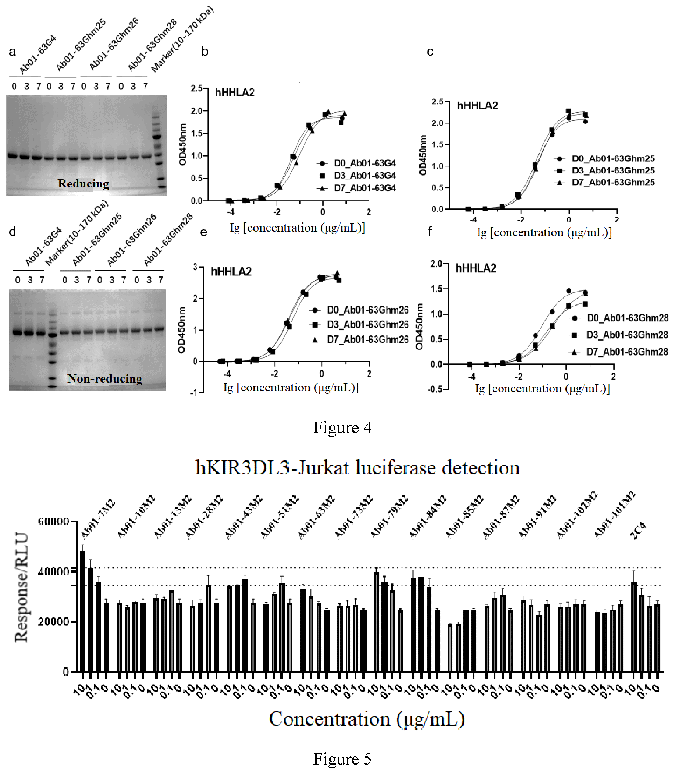

FIG. 4 shows SDS-PAGE and ELISA results of some HHLA2 antibodies of the present application, where FIG. 4a shows SDS-PAGE reducing electrophoresis results of a candidate parental antibody Ab01-63G4 and 3 humanized or engineered mutants thereof after being treated at 37°C for 0, 3, and 7 days, FIG. 4b shows binding curves of the candidate parental antibody Ab01-63G4 to human HHLA2 after being treated at 37°C for 0, 3, and 7 days, FIG. 4c shows binding curves of a mutant Ab01-63Ghm25 to human HHLA2 after being treated at 37°C for 0, 3, and 7 days, FIG. 4d shows SDS-PAGE non-reducing electrophoresis results of the candidate parental antibody Ab01-63G4 and 3 humanized or engineered mutants thereof after being treated at 37°C for 0, 3, and 7 days, FIG. 4e shows binding curves of a mutant Ab01-63Ghm26 to human HHLA2 after being treated at 37°C for 0, 3, and 7 days, and FIG. 4f shows binding curves of a mutant Ab01-63Ghm28 to human HHLA2 after being treated at 37°C for 0, 3, and 7 days.
FIG. 5 shows results of some HHLA2 antibodies of the present application blocking HHLA2/KIR3DL3 signaling pathway.

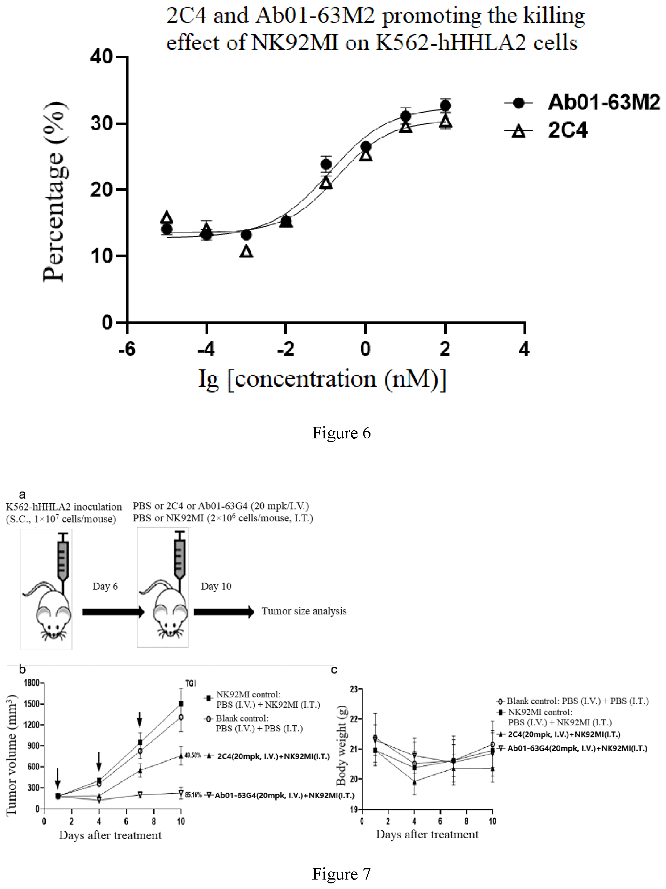

FIG. 6 shows results of some HHLA2 antibodies of the present application promoting the killing effects of NK92MI on target cells.
FIG. 7 shows results of anti-tumor effects of some HHLA2 antibodies of the present application in the NCG mouse/NK92MI/K562-hHHLA2 CDX model, where FIG. 7a shows an experimental protocol, FIG. 7b shows tumor growth curves of different groups, and FIG. 7c shows body weight change curves of mice in different groups.

### DETAILED DESCRIPTION

Technical solutions of the present application are further described below through examples. It is to be understood by those skilled in the art that the examples described below are intended to facilitate understanding of the present application and are not to be construed as limiting the present application.

Experiments without specific techniques or conditions specified in the examples are conducted according to techniques or conditions described in the literature in the art or product specifications. The reagents or instruments used herein, without manufacturers specified, are conventional products commercially available from proper channels.

### Sequence Listing

| Name of Antibody | Full-Length Sequence SEQ ID NO: | Variable Region Sequence SEQ ID NO: | CDR1 (Kabat) SEQ ID NO: | CDR2 (Kabat) SEQ ID NO: | CDR3 (Kabat) SEQ ID NO: |
|---|---|---|---|---|---|
| Ab01-63Ghm25 | 1 | 15 | 29 | 37 | 47 |
| Ab01-63Ghm28 | 2 | 16 | 29 | 37 | 48 |
| Ab01-63Ghm26 | 3 | 17 | 29 | 38 | 47 |
| Ab01-63Ghm35 | 4 | 18 | 29 | 37 | 49 |
| Ab01-63Ghm36 | 5 | 19 | 29 | 37 | 50 |
| Ab01-63G4 | 6 | 20 | 29 | 39 | 47 |
| Ab01-63M2 | 7 | 20 | 29 | 39 | 47 |
| Ab01-7M2 | 8 | 22 | 30 | 40 | 51 |
| Ab01-84M2 | 9 | 23 | 31 | 41 | 52 |
| Ab01-91M2 | 10 | 24 | 32 | 42 | 53 |
| Ab01-121M1 | 11 | 25 | 33 | 43 | 54 |
| Ab01-122M1 | 12 | 26 | 34 | 44 | 55 |
| Ab01-126M1 | 13 | 27 | 35 | 45 | 55 |
| Ab01-110M1 | 14 | 28 | 36 | 46 | 56 |

### Example 1

### Screening of anti-human HHLA2 nanobody molecules by phage display

Alpacas were immunized with an emulsified mixture of 500 µg of human HHLA2 proteins (Acro Biosystems, B77-H52H5) and 250 µL of Freund's complete adjuvant, once every two weeks, for a total of 4 times. Peripheral blood was collected before antigen immunization and after each immunization to obtain serum. About one week after the fourth immunization, blood was collected, and an antibody titer against the immunizing antigen in the peripheral serum after each immunization was detected. An antibody titer reaching 10⁵ indicated an immunization success. Then, 50 mL of peripheral blood of an alpaca was collected, and peripheral blood mononuclear cells (PBMCs) were isolated for immune library construction.

RNA in the PBMCs (from 50 mL of peripheral blood of the alpaca) was extracted by the Trizol method, and concentration and gel electrophoresis detections were performed to analyze the quality of the total RNA. The extracted total RNA was divided into two equal parts, and the two equal parts were subjected to reverse transcription using oligo(dT) and Random primers respectively to obtain cDNA. With cDNA obtained through reverse transcription with different primers as templates, two rounds of PCR were performed to obtain PCR products of diversified variable region fragments of alpaca heavy-chain antibodies, that is, VHH DNA sequences. Purified PCR products of the diversified VHH DNA sequences were digested with restriction enzymes SpeI (Takara, 1086A) and SacI (Takara, 1078A) and were ligated and inserted into phagemid vectors pComb3XSS with T4 ligase (NEB, M0202L). Recombinant phagemids were transformed into *Escherichia coli* TG1 through electroporation, plated, and cultured overnight. Bacterial cells were collected to obtain a bacterial library. Diversified phages in the bacterial library were amplified and purified to obtain a phage library for subsequent screening.

The alpaca-derived phage display nanobody library was screened by an immunotube method, wherein the capacity of the selected phage display library was 2×10⁹. The target protein was coated on immunotubes at a concentration of 20 µg/mL and subjected to three rounds of enrichment screening. The enrichment degrees of all the rounds of screening are listed in Table 1.

**Table 1**

| Screening Round No. | Input Titer | Screening Titer | Control Titer | Enrichment Degree | Fold |
|---|---|---|---|---|---|
| Round 1 | 1.0× 10¹² pfu | 2.0×10⁹ pfu/mL | 1.0×10⁸ pfu/mL | 2.0×10⁻³ | 20 |
| Round 2 | 2.5×10¹² pfu | 3.6×10¹⁰ pfu/mL | 4.8×10⁷ pfu/mL | 1.44×10⁻² | 750 |
| Round 3 | 8.0×10¹² pfu | 6.4×10¹¹ pfu/mL | 2.8×10⁸ pfu/mL | 8.0×10⁻² | 2285 |

During the third round of phage panning, phages were eluted for 30 min by rotating at room temperature using a 0.25 mg/mL Trypsin solution, or a 50 µg/mL KIR3DL3 (Acro Biosystems, KI3-H5259) solution, or a 5 µg/mL solution of a positive control antibody 2C4 (prepared by Artivila Biopharma and including sequences from CN112153977A, where a VH sequence is shown in SEQ ID NO: 57 and a VL sequence is shown in SEQ ID NO: 58). The third-round phage eluate was used to infect *Escherichia coli* TG1. After serial dilution and plating, plates were placed in a 32°C incubator overnight. Single clone colonies were picked for phage amplification and then verified by ELISA (coating antigen: human HHLA2). The corresponding backup positive clone TG1 colonies were subjected to nanobody gene sequencing. Through sequence analysis, 136 different anti-human HHLA2 nanobody molecules were preliminarily obtained.
SEQ ID NO: 57: 
EVHLQQSGPELEKPGVSVKISCKASGFSFTDYNMNWVKQSSGKSLEWIGNIDPYYG
RINYNQKFKGKATLSVDKSSSTAYMHLKSLTSEDSAVYYCATGAYTSGYSWFAYWGQGT
LVTVSS.
SEQ ID NO: 58:

ENVLTQSPGIMAASLGEKVTMTCSASSSVSSSYLHWYQQRSGASPKPLIHRTSNLAS
GVPARFSGSGSGTSYSLTISSVEAEDDATYYCQQWSGYPFTFGSGTKLEIK.

### Example 2

### Antibody expression and purification

Codon-optimized nucleotide sequences of the nanobody molecules obtained in Example 1 were synthesized and cloned into eukaryotic expression vectors pcDNA3.1-hG1Fc or pcDNA3.1-mG2aFc designed and modified in our laboratory (where an Fc region of human IgG1 or mouse IgG2a has been inserted into each vector). Expression plasmids were prepared using the Tiangen Endotoxin-Free Plasmid Extraction Kit and transfected into ExpiCHO cells using the ExpiFectamine CHO Transfection kit (Gibco, A29130). Then, cell culture flasks were placed in a shaker and cultured for 7 days at 37°C in 8% CO₂ at 100 rpm. The cell culture supernatants containing the target proteins were collected through centrifugation, and target antibodies were purified through affinity chromatography using the rProtein A Beads 4FF filler (Changzhou Smart-Lifesciences Biotechnology Co., Ltd., SA015025). The target antibodies were eluted with a 0.1 M glycine solution with a pH of 3.5 and concentrated and exchanged into PBS buffer using ultrafiltration tubes (SHENZHEN IVDTIMES, MCP030C41). After concentration determination by A280 and purity detection through protein electrophoresis, the antibodies were aliquoted and cryopreserved for later use. Table 2 lists the transient expression data of some antibodies. FIG. 4 shows SDS-PAGE results of a candidate parental antibody Ab01-63G4 and 3 humanized or engineered mutants thereof (Ab01-63Ghm25, Ab01-63Ghm26, and Ab01-63Ghm28) after being treated at 37°C for 0, 3, and 7 days (FIG. 4a shows reducing electrophoresis, and FIG. 4d shows non-reducing electrophoresis). It can be seen that the candidate parental antibody Ab01-63G4 and the 3 humanized or engineered mutants thereof (Ab01-63Ghm25, Ab01-63Ghm26, and Ab01-63Ghm28) have good purity and stability.

**Table 2**

| Name of Antibody | SEQ ID NO: | Expression Volume (mL) | Expression Amount (g/L) |
|---|---|---|---|
| Ab01-63Ghm25 | 1 | 25 | 119 |
| Ab01-63Ghm28 | 2 | 25 | 169 |
| Ab01-63Ghm26 | 3 | 25 | 64 |
| Ab01-63Ghm35 | 4 | 25 | 29 |
| Ab01-63Ghm36 | 5 | 25 | 52 |
| Ab01-63G4 | 6 | 150 | 201 |
| Ab01-63M2 | 7 | 25 | 145 |
| Ab01-7M2 | 8 | 25 | 253 |
| Ab01-84M2 | 9 | 25 | 183 |
| Ab01-91M2 | 10 | 25 | 132 |
| Ab01-121M1 | 11 | 25 | 348 |
| Ab01-122M1 | 12 | 25 | 206 |
| Ab01-126M1 | 13 | 25 | 137 |
| Ab01-110M1 | 14 | 25 | 137 |
| Ab01-10M2 | - | 25 | 163 |
| Ab01-13M2 | - | 25 | 206 |
| Ab01-28M2 | - | 25 | 170 |
| Ab01-43M2 | - | 25 | 222 |
| Ab01-51M2 | - | 25 | 165 |
| Ab01-63M2 | - | 25 | 145 |
| Ab01-73M2 | - | 25 | 187 |
| Ab01-79M2 | - | 25 | 203 |
| Ab01-85M2 | - | 25 | 124 |
| Ab01-87M2 | - | 25 | 187 |
| Ab01-101M2 | - | 25 | 117 |
| Ab01-102M2 | - | 25 | 122 |

Ab01-10M2 to Ab01-102M2 are all antibodies with known sequences in the existing art.

### Example 3

### ELISA screening of antibodies capable of binding to human HHLA2

0.5 µg/mL human HHLA2 proteins (Acro Biosystems, B77-H52H5) were coated on the Immuno transparent standard plate (ThermoFisher, 468667), at a volume of 100 µL per well, and incubated overnight at 4°C. The plate was washed with PBST, added with a PBS blocking solution containing 1% BSA, 200 µL per well, and blocked for 1 h at 37°C. Antibody proteins prepared in Example 2 were added at different concentrations and incubated for 1 h at 37°C. The plate was washed with PBST, added with the Goat anti-Human IgG-Fc Secondary Antibody (HRP, Invitrogen, A18817) labeled with horseradish peroxidase, and incubated for 30 min at 37°C. The plate was washed 5 times with PBST, added with TMB (Solarbio, PR1200), 50 µL per well, and placed at room temperature (20±5°C) in the dark for 10 min. Then, 50 µL of ELISA stop solution (Solarbio, C1058) was added to each well to terminate the substrate reaction. The OD value was read at 450 nm with a microplate reader, and an ability of an antibody to bind to human HHLA2 was analyzed.

The candidate antibody molecules Ab01-63M2, Ab01-7M2, Ab01-84M2, Ab01-91M2, Ab01-121M1, Ab01-122M1, Ab01-126M1, and Ab01-110M1 can all bind to human HHLA2 as verified by ELISA. The ELISA binding curves of some HHLA2 antibodies to human HHLA2 are shown in FIG. 1 (FIG. 1a and FIG. 1b) and FIG. 4 (FIG. 4b, FIG. 4c, FIG. 4e, and FIG. 4f). It can be seen that the candidate parental antibody Ab01-63G4 and the 3 humanized or engineered mutants thereof (Ab01-63Ghm25, Ab01-63Ghm26, and Ab01-63Ghm28) exhibit relatively high affinity to human HHLA2.

### Example 4

### FACS screening of antibodies capable of specifically binding to human/cynomolgus monkey HHLA2

A K562 cell line (Pricella), a K562 cell strain overexpressing human HHLA2 (K562-hHHLA2, Artivila Biopharma), an HEK293 cell line (Zhuhai Kairui), an HEK293 cell strain overexpressing cynomolgus monkey HHLA2 (HEK293-cyHHLA2, Artivila Biopharma), and an HCC827 cell line highly expressing human HHLA2 (Pricella) were used for antibody binding experiments at a cellular level. Various cells were collected using 1× PBS, centrifuged at 300 g for 5 min, and resuspended in a blocking buffer (PBS + 5% FBS) pre-cooled on ice. The cell density was adjusted to 10⁶/mL. 50 µL of cells and 50 µL of a serially diluted antibody to be tested were added to each well of a 96-well V-bottom plate (Corning, 3894) and incubated for 2 h on ice in the dark. 200 µL of a wash buffer (PBS + 0.1% BSA) pre-cooled on ice was added and centrifuged at 300 g for 5 min, and the supernatant was carefully discarded, which were repeated once. The cells were resuspended in a blocking buffer (PBS + 5% FBS) containing a 500-fold diluted fluorescent secondary antibody (APC anti-human IgG Fc, BioLegend, 409306 or Alexa Fluor^{®} 647-AffiniPure Goat Anti-Mouse IgG Fc, Jackson, 115-605-164) and incubated for 30 min on ice in the dark. 200 µL of a wash buffer (PBS + 0.1% BSA) pre-cooled on ice was added and centrifuged at 300 g for 5 min, and the supernatant was discarded, which were repeated once. The cells were resuspended in 100 µL of PBS and placed on ice. Samples were transferred to flow cytometry tubes, and the corresponding fluorescence signals were detected using a flow cytometer (Wellgrow Easy Cell 206A1).

The positive control antibody 2C4 and the candidate antibody molecules Ab01-63M2, Ab01-7M2, Ab01-84M2, Ab01-91M2, Ab01-121M1, Ab01-122M1, Ab01-126M1, and Ab01-110M1 can all specifically bind to human/cynomolgus monkey HHLA2 as verified by FACS. The results of specific binding of some HHLA2 antibodies to human/cynomolgus monkey HHLA2 (FACS) are shown in FIG. 2. The results of binding of some HHLA2 antibodies to HCC827 tumor cells (FACS) are shown in FIG. 3. It can be seen that the candidate parental antibody Ab01-63M2 and the positive control antibody 2C4 can both bind to human/cynomolgus monkey HHLA2 at the cellular level and can bind to the human non-small-cell lung carcinoma cell line HCC827 highly expressing HHLA2.

**Table 3**

| | Ab01-63M2 | 2C4 |
|---|---|---|
| EC₅₀ (nM) | 0.043 | 0.281 |

EC₅₀ in Table 3 corresponds to the results of binding of the HHLA2 antibodies to HCC827 tumor cells (FACS). As can be seen from the data in Table 3, the ability of the candidate parental antibody Ab01-63M2 to bind to the tumor cell line HCC827 is slightly better than that of the positive control antibody 2C4.

### Example 5

### Screening of antibodies that can block the binding of HHLA2/KIR3DL3 through luciferase reporter gene assay

Four treatments were arranged as shown in the following table:

**Table 4**

| No. | Group Name |
|---|---|
| 1 | K562-hHHLA2 + hKIR3DL3-Jurkat + antibody to be tested + OKT3 |
| 2 | K562-hHHLA2 + hKIR3DL3-Jurkat + cell culture medium + OKT3 |
| 3 | K562 + hKIR3DL3-Jurkat + cell culture medium + OKT3 |
| 4 | K562 + hKIR3DL3-Jurkat + cell culture medium |

The cell density of K562 or K562-hHHLA2 was adjusted to 4×10⁵ cells/mL. The cells were transferred to a white opaque bottom 96-well plate (ThermoFisher, 15042) (25 µL/well) and added with an equal volume of a serially diluted antibody to be tested or a cell culture medium (RPMI1640 + 10% FBS + 1% P/S) and pre-incubated for 30 min. The cell density of hKIR3DL3-Jurkat (overexpressing human KIR3DL3 proteins, Genomeditech) was adjusted to 1×10⁶ cells/mL. The cells were added to the above 96-well plate (50 µL/well). 25 µL of an anti-CD3 epsilon antibody OKT3 (Genomeditech, GM-51478AB-100) was added to a final concentration of 0.2 µg/mL and incubated for 30 min. The cells were incubated for 16 h in a 37°C CO₂ incubator. The fluorescence signals were determined using the GMOne-Step Luciferase Reporter Gene Assay Kit (Genomeditech, GM-040403B).

The results of the luciferase reporter gene assay show that the positive control 2C4 and the candidate antibody molecules Ab01-63M2, Ab01-7M2, Ab01-84M2, Ab01-91M2, Ab01-121M1, Ab01-122M1, Ab01-126M1, and Ab01-110M1 can all block the HHLA2/KIR3DL3 signaling pathway. The results of some HHLA2 antibodies blocking HHLA2/KIR3DL3 are shown in FIG. 5. It can be seen that the candidate antibodies Ab01-7M2, Ab01-43M2, Ab01-63M2, Ab01-79M2, and Ab01-84M2 have better or similar abilities to block HHLA2/KIR3DL3 compared with the positive control antibody 2C4.

### Example 6

### Detection of activation effects of anti-HHLA2 antibodies on NK92MI cells by LDH method

Five treatments were arranged as shown in the following table:

**Table 5**

| No. | Group Name | Sample Addition Settings (3 duplicate wells) |
|---|---|---|
| 1 | Group of co-incubation of effector cells and target cells + antibody (ER1) | 100 µL of effector cells + 50 µL of an antibody to be tested + 50 µL of target cells |
| 2 | Group of effector cells alone (ESR1) | 100 µL of effector cells + 100 µL of N500 culture medium |
| 3 | Group of target cells alone (TSR) | 50 µL of target cells + 150 µL of N500 culture medium |
| 4 | Maximum release group of target cells (TMR) | 50 µL of target cells+ 100 µL of N500 culture medium + 50 µL of 10% Triton X-100 (added before or after incubation) |
| 5 | Medium release group (CMB) | 200 µL of N500 culture medium |

K562 or K562-hHHLA2 cells were collected. The density was adjusted to 4×10⁵ cells/mL with the N500 serum-free culture medium (Dakewe, 6113031). The cells were added to a V-shaped/bottom 96-well plate (50 µL/well), added with a serially diluted antibody to be tested (50 µL/well), and pre-incubated for 30 min. Effector cells, NK92MI cells (Pricella, CL-0533, KIR3DL3-positive effector cells), were collected. The density was adjusted to 2×10⁵ cells/mL with the N500 culture medium. The NK92MI cells were added to the above cells or culture medium (100 µL/well), centrifuged at 250 g for 3 min, and incubated for 6 h at 37°C. The culture medium was centrifuged at 250 g for 5 min at normal temperature. 50 µL of the supernatant was aspirated into a 96-well plate. The cell killing results were determined using the LDH cytotoxicity detection kit (Roche, 11644793001).

The LDH cell killing results show that the positive control 2C4 and the candidate antibody molecules Ab01-63M2, Ab01-7M2, Ab01-84M2, Ab01-91M2, Ab01-121M1, Ab01-122M1, Ab01-126M1, and Ab01-110M1 can all significantly enhance the killing effect of NK92MI cells on the target cells K562-hHHLA2. The results of some HHLA2 antibodies promoting the killing effects of NK92MI on target cells are shown in FIG. 6.

**Table 6**

| | 2C4 | Ab01-63M2 |
|---|---|---|
| EC₅₀ (nM) | 0.1922 | 0.1251 |

EC₅₀ in Table 6 corresponds to the results of the HHLA2 antibodies 2C4 and Ab01-63M2 promoting the killing effects of NK92MI on target cells (K562 overexpressing human HHLA2) in FIG. 6. As can be seen from the data shown in Table 6, the candidate parental antibody Ab01-63M2 and the positive control antibody 2C4 have comparable abilities to promote the killing effects of NK92MI on K562-hHHLA2.

### Example 7

### Engineering of candidate antibodies

As is known to those skilled in the art, alpaca nanobodies generally have relatively low immunogenicity. Since the germline genes of the alpaca nanobodies are highly homologous to human germline genes, the humanization operation process of the alpaca nanobodies is relatively simple and generally includes conventional CDR grafting (if a humanized antibody has a reduced antigen binding ability, back mutants may be attempted on the corresponding differential sites). Optionally, retaining 4 conserved amino acids in a framework region (FR) of a nanobody is generally beneficial for improving the hydrophilicity and solubility of humanized nanobody molecules. Specifically, for example, to humanize the candidate antibody molecule Ab01-63M2 (SEQ ID NO: 7) or Ab01-63G4 (SEQ ID NO: 6), the IMGT online database may be used for alignment to select a human germline gene IGHV3_23+IGHJ5 with the highest homology to the germline gene of a variable region (SEQ ID NO: 20) of the candidate nanobody as a framework. After CDR grafting and substitution of 4 conserved amino acids in the framework region (FR) (Kabat numbering: V37F, G44E, L45R, W47G), a preliminary humanized nanobody sequence (SEQ ID NO: 21) may be obtained. Further, sequence analysis may be performed on PTM modification sites: a mutant N74T may be made to SEQ ID NO: 21 to eliminate the aspartate isomerization and asparagine deamidation motif "D73N74S75" in the FR3 region; secondly, several amino acid substitutions may be made to high-risk aspartate isomerization sites D55 and D62 in CDR2 of SEQ ID NO: 21. Furthermore, based on three-dimensional structural prediction analysis, several amino acid substitutions may be made for mutational optimization on a positively charged patch "R99R100" and a hydrophobic patch "F112F113F114" in CDR3, to obtain an antibody molecule with better druggability. Optionally, as is known to those skilled in the art, corresponding engineering on the Fc region of the antibody can silence or enhance an Fc effector function or prolong or shorten a half-life.

The optimized druggability of the antibody molecule may generally be embodied in improvements in one or more aspects, such as increased antibody stability, optimized affinity (either increased or decreased), an increased yield, increased solubility, and increased anti-tumor activity. After the stability, affinity, yield, purity, activity, and other aspects of a series of engineered mutants of Ab01-63M2 or Ab01-63G4 are tested and compared, preferred antibody molecules are Ab01-63Ghm25 (SEQ ID NO: 1), Ab01-63Ghm28 (SEQ ID NO: 2), Ab01-63Ghm26 (SEQ ID NO: 3), Ab01-63Ghm35 (SEQ ID NO: 4), and Ab01-63Ghm36 (SEQ ID NO: 5).

FIG. 1a and FIG. 1b show ELISA binding curves of the candidate parental antibody Ab01-63G4 and the humanized or engineered mutants thereof to human HHLA2. FIG. 4 shows the SDS-PAGE results of the candidate parental antibody Ab01-63G4 and 3 humanized or engineered mutants thereof (Ab01-63Ghm25, Ab01-63Ghm26, and Ab01-63Ghm28) after being treated at 37°C for 0, 3, and 7 days and their ELISA binding curves to human HHLA2. It can be seen that compared with the candidate parental antibody Ab01-63G4, the 3 humanized or engineered mutants Ab01-63Ghm25, Ab01-63Ghm26, and Ab01-63Ghm28 exhibit similar affinity and increased stability.

### Example 8

### Determination of anti-tumor efficacy of candidate antibodies in NCG mouse/NK92MI/K562-hHHLA2 CDX model

NCG mice were used to establish a K562-hHHLA2 CDX tumor model for *in vivo* pharmacodynamic studies. The specific method was as follows: according to experimental requirements, K562-hHHLA2 cells were revived and expanded in culture, and tumor cells were inoculated at 1×10⁷ cells/mouse. When the average tumor volume of the mice reached 100-200 mm³, the mice were grouped and administered, once every three days, for a total of three times via tail vein injection, where an administration regimen was 20 mpk/I.V./Q3D. 2 h after antibody administration, intratumoral injection of NK92MI cells was performed, 2×10⁶ cells injected each time. After the first administration, the body weight and tumor volume were measured every three days. The tumor volume was calculated: tumor volume (mm³) = 0.5 × tumor length × (tumor width)². The experiment was terminated on day 10 after administration. All the mice were euthanized.

The *in vivo* anti-tumor effects in the NCG/K562-hHHLA2 CDX tumor model are shown in FIG. 7. Specifically, on day 10 after administration, the mice in the positive antibody (2C4 whose variable regions have sequences shown in SEQ ID NO: 57-58 and from the published patent CN112153977A) control group have an average tumor volume of 757.5 mm³ and a tumor growth inhibition (TGI) of 49.58%; on day 10 after administration, the candidate antibody Ab01-63G4 treatment group has an average tumor volume of 223.0 mm³ (exhibiting significant differences (p < 0.05) compared with the vehicle control group and the positive antibody control group) and a tumor growth inhibition (TGI) of 85.16%. Thus, Ab01-63G4 (20 mpk/I.V./Q3D) exhibits significant efficacy in the K562-hHHLA2 NCG CDX model and is superior to the positive control 2C4.

The applicant has stated that although the anti-human HHLA2 nanobody and the use thereof in the present application are described through the preceding embodiments, the present application is not limited to the preceding embodiments, which means that the implementation of the present application does not necessarily depend on the preceding embodiments. It is to be understood by those skilled in the art that any improvements made to the present application and equivalent replacements of raw materials of the product, additions of adjuvant ingredients, selections of specific manners, etc. in the present application all fall within the scope of the present application.

Although preferred embodiments of the present application are described above in detail, the present application is not limited to details of the preceding embodiments, and various simple variations can be made to the technical solutions of the present application without departing from the technical concept of the present application. These simple variations are all within the scope of the present application.

Additionally, it is to be noted that if not in conflict, specific technical features described in the preceding embodiments can be combined in any suitable manner. To avoid unnecessary repetition, various possible combinations are no longer described in the present application.

## Claims

1. An anti-human HHLA2 nanobody, which has a variable region comprising CDR1, CDR2, and CDR3, wherein an amino acid sequence of CDR1 comprises any one of the sequences shown in SEQ ID NO: 29 to SEQ ID NO: 36 or a mutant thereof, an amino acid sequence of CDR2 comprises any one of the sequences shown in SEQ ID NO: 37 to SEQ ID NO: 46 or a mutant thereof, an amino acid sequence of CDR3 comprises any one of the sequences shown in SEQ ID NO: 47 to SEQ ID NO: 56 or a mutant thereof, wherein the mutant refers to an amino acid sequence having a substitution, insertion, or deletion of 1, 2, 3, or 4 amino acids.

2. The anti-human HHLA2 nanobody according to claim 1, wherein the amino acid sequence of CDR1 is the sequence shown in SEQ ID NO: 29 or SEQ ID NO: 31.

3. The anti-human HHLA2 nanobody according to claim 1, wherein the amino acid sequence of CDR2 is the sequence shown in SEQ ID NO: 37 or SEQ ID NO: 38.

4. The anti-human HHLA2 nanobody according to claim 1, wherein the amino acid sequence of CDR3 is the sequence shown in any one of SEQ ID NO: 48 to SEQ ID NO: 50.

5. The anti-human HHLA2 nanobody according to claim 1, wherein the nanobody comprises a single-domain antibody, a VHH-Fc antibody, a bispecific antibody, or a multispecific antibody;
preferably, the nanobody comprises a variable region, a fragment crystallizable region of a heavy chain or a mutant thereof;
preferably, the fragment crystallizable region of the heavy chain is selected from any one of human IgG1 or a mutant thereof, human IgG2 or a mutant thereof, human IgG3 or a mutant thereof, or human IgG4 or a mutant thereof; or
the fragment crystallizable region of the heavy chain is selected from any one of murine IgG1 or a mutant thereof, murine IgG2a or a mutant thereof, murine IgG2b or a mutant thereof, or murine IgG3 or a mutant thereof;
preferably, the fragment crystallizable region of the heavy chain is selected from any one of human IgG1 or a mutant thereof, or murine IgG2a or a mutant thereof;
preferably, an amino acid sequence of the variable region in the nanobody comprises the sequence shown in any one of SEQ ID NO: 15 to SEQ ID NO: 28;
preferably, an amino acid sequence of the VHH-Fc antibody comprises the sequence shown in any one of SEQ ID NO: 1 to SEQ ID NO: 14.

6. A nucleic acid molecule encoding the anti-human HHLA2 nanobody according to any one of claims 1 to 5.

7. An expression vector, comprising the nucleic acid molecule according to claim 6.

8. A host cell, comprising the nucleic acid molecule according to claim 6 or the expression vector according to claim 7.

9. A chimeric antigen receptor, comprising the anti-human HHLA2 nanobody according to any one of claims 1 to 5.

10. A genetically modified cell, comprising the chimeric antigen receptor according to claim 9.

11. A bispecific or multispecific antibody, comprising the anti-human HHLA2 nanobody according to any one of claims 1 to 5.

12. A conjugate, comprising the anti-human HHLA2 nanobody according to any one of claims 1 to 5.

13. A pharmaceutical composition, comprising the anti-human HHLA2 nanobody according to any one of claims 1 to 5.

14. Use of the anti-human HHLA2 nanobody according to any one of claims 1 to 5, the nucleic acid molecule according to claim 6, the expression vector according to claim 7, the bispecific or multispecific antibody according to claim 11, or the conjugate according to claim 12 in preparation of a product for detecting human HHLA2 proteins.

15. Use of the anti-human HHLA2 nanobody according to any one of claims 1 to 5, the nucleic acid molecule according to claim 6, the expression vector according to claim 7, the chimeric antigen receptor according to claim 9, the genetically modified cell according to claim 10, the bispecific or multispecific antibody according to claim 11, the conjugate according to claim 12, or the pharmaceutical composition according to claim 13 in preparation of a medicament for treating and/or preventing cancer.
